# EUROPEAN PATENT SPECIFICATION

(11) **EP 3 700 509 B1**
(45) Date of publication and mention of the grant of the patent: **22.11.2023**
(21) Application number: 17916454.6
(22) Date of filing: 19.12.2017
(51) Int. Cl.: A61K 9/28, A61K 31/167, A61K 31/192, A61P 29/00

(54) **COLOR STABLE FIXED DOSE TABLET COMBINATION OF IBUPROFEN AND PARACETAMOL**
FARBSTABILE FEST DOSIERTE TABLETTENKOMBINATION AUS IBUPROFEN UND PARACETAMOL
ASSOCIATION D'IBUPROFÈNE ET DE PARACÉTAMOL EN COMPRIMÉS À DOSES FIXES ET À COULEUR STABLE

(30) Priority: 29.11.2017 TR 201719166
(43) Date of publication of application: 02.09.2020
(73) Proprietor: Ilko Ilaç Sanayi Ve Ticaret Anonim Sirketi, Sancaktepe/Istanbul (TR)
(72) Inventor: ÖNCEL, Hatice, Sancaktepe/Istanbul (TR); ÇAPAN, Yilmaz, 06800 Çankaya/Ankara (TR); PINARBASLI, Onur, 06800 Çankaya/Ankara (TR); YURTOGLU, Sibel, 06800 Çankaya/Ankara (TR); SARRAÇOGLU, Nagehan, 06800 Çankaya/Ankara (TR)
(74) Representative: Bulut, Pinar
(86) International application number: PCT/TR2017/050678
(87) International publication number: WO 2019/108147

(56) References cited:
- WO-A2-2006/004449
- US-A- 5 409 709
- Anonymous: "REVISION OF MONOGRAPH ON TABLETS Final text for addition to The International Pharmacopoeia", , 1 March 2011 (2011-03-01), pages 1-10, XP055557373, Retrieved from the Internet: URL:https://www.who.int/medicines/publicat ions/pharmacopoeia/Tabs-GeneralMono-rev-FI NAL_31032011.pdf [retrieved on 2019-02-15]

## Description

### Technical field:

The present invention relates to an oral pharmaceutical tablet composition comprising paracetamol and ibuprofen or pharmaceutically acceptable salts thereof wherein the said tablet formulation has color stable tablet properties upon heat and humidity stability conditions, wherein the pharmaceutical tablet formulation is characterized in that said tablet comprises a sub-coating layer wherein this layer comprising a high-chain (6 to 12 carbons) fatty acid esters of glycerol which is glyceryl monocaprylocaprate with a content of 0.1 %-0.3% (w/w) by weight of the tablet composition.

### Prior Art:

Paracetamol (also known as acetaminophen) is a non-opiate, non-salicylate analgesic and antipyretic drug with a molecular formula C₈H₉NO₂. Its chemical name is N-(4-hydroxyphenyl) ethanamide*N*-(4-hydroxyphenyl) acetamide (N-acetyl-para-aminophenol (APAP)) and it is represented by Formula I (shown below);

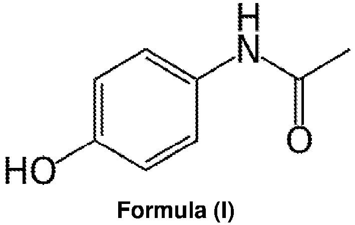

Paracetamol is typically used for mild to moderate pain asa peripherally acting analgesic. It produces analgesia by elevation of the pain threshold and antipyresis through action on the hypothalamic heat-regulating center. It provides temporary relief of minor aches and pains with heartburn or acid indigestion and upset stomach associated with these symptoms.

Ibuprofen is a nonsteroidal anti-infammatory drug (NSAID) class that is used for treating pain, fever and inflammation, possesses analgesic and antipyretic activities. Its molecular formula is C₁₃H₁₈O₂, chemical name of which is (RS)-2-(4-(2-Methylpropyl) phenyl) propanoic acid. The structural formula of Ibuprofen is represented by Formula II (shown below);

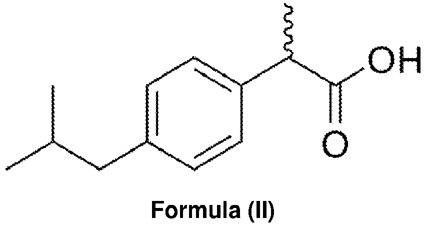

Ibuprofen mode of action is related to prostaglandin synthetase inhibition. It is indicated in the treatment for relief of the signs and symptoms of rheumatoid arthritis and osteoarthritis, mild to moderate pain and treatment of primarydysmenorrhea. Combination of ibuprofen and paracetamol is used for the temporary relief of mild to moderate pain associated with migraine, headache, backache, period pain, dental pain, rheumatic and muscular pain, pain of non-serious arthritis, cold and flu symptoms, sore throat and fever.

The tablet dosage form of paracetamol and ibuprofen are commercially marketed under the trade name of Nuromol^{®} (Reckitt Benckiser Healthcare (UK)) (Ibuprofen 200 mg and Paracetamol 500 mg).

WO 2006/004449 describes a combination pharmaceutical composition for the treatment of pain including about 125 mg to about 150 mg ibuprofen and about 475 mg to about 500 mg paracetamol.

WO 2007/034135 describes a process for producing a granular composition comprising a plurality of solidified melt granules including a nonsteroidal antiinflammatory drug (NSAID) and paracetamol.

Non-steroidal anti-inflammatory drugs (NSAIDs) are a widely used class of medicaments. They are a well-defined group of compounds and include phenylpropionic acids such as ibuprofen, naproxen, ketoprofen and flurbiprofen. They are generally acidic and substantially insoluble drugs and also conveniently administered as an oral pharmaceutical composition in the form of tablets.

Hyllested, M et. al (British Journal of Anaesthesia (2002), 88(2): 199-214) have reported that the addition of an NSAID to paracetamol may confer additional analgesic efficacy compared with paracetamol alone, and also suggest that paracetamol may enhance analgesia when added to an NSAID, compared with NSAIDs alone.

Since many of the NSAIDs are acidic drugs, accordingly, absorption can be a problem in the acidic conditions encountered in the stomach. Furthermore, although the literature has proposed many formulations adapted to disintegrate quickly, a major problem occurs with ibuprofen and other NSAIDs as they may need to be administered in relatively high doses, e.g. up to 800 mg per unit dose. Thus, there is a problem to provide a dosage form which includes the NSAID together with excipients useful to formulate the tablet into the dosage form and also excipients useful to ensure rapid disintegration, but not to provide a tablet that is too large for patient consumption or cannot be produced according to standard large scale manufacturing processes. Furthermore, the solid dosage form must be sufficiently hard to withstand the rigours of the manufacturing process (for example as encountered during the stage of film coating in a perforated rotating drum and packaging etc.) but must have appropriate disintegration characteristics to ensure rapid release of the drug from the formulation and also appropriate dissolution characteristics.

It has been recognized that solid dosage forms such as tablets containing ibuprofen and other ingredients tend to exhibit stability problems, including the formation of low melting point eutectics. In general, eutectic compositions often exhibit unique physical properties such as melting or freezing points which are not characteristic of the individual constituents.

US5512300 states that, it has been found that certain ibuprofen-containing solid dosage forms such as powders, granules, pellets, capsules, tablets and the like exhibit stability problems. It is further recognized that the instability of such dosage forms is at least in part attributable to the tendency of these formulations to undergo undesirable melting point depressions. For example, although the melting point of ibuprofen is approximately 75°C, formulations containing ibuprofen and terfenadine melt at temperatures ranging from about 50°C to about 75°C. This melting point depression phenomena is thought to be attributable to the tendency of ibuprofen to form eutectic compositions with certain substances. Ibuprofen also forms low melting point eutectics with substances such as diphenhydramine hydrochloride and astemizole.

Eutectic formations, however, significantly reduce the shelf-life of ibuprofen-containing products. For example, many prior art tablets containing ibuprofen and other active ingredients and/or excipients tend to exhibit structural deteriorations such as crumbling, pitting and fissuring, as well as a loss of color as a result of the eutectic formation. Alterations in the crystalline structures have also been observed.

According to US Patent 5512300, some ibuprofen-containing formulations tend to exhibit melting point depressions almost immediately. Other formulations are stable initially but undergo changes in consistency upon exposure to fluctuations in temperature and/or other environmental factors. Still other ibuprofen-containing formulations may inherently develop these melting point depressions upon prolonged intimate contact of the ibuprofen with the other constituents present in the formulations. Each of these situations creates storage problems.

US 5512300 discloses a method of stabilizing ibuprofen for use in a composition comprising: directly heating ibuprofen to form a melt; combining said ibuprofen melt with an alkali metal to forge an amalgamation; and mixing the ibuprofen melt with the alkali metal to form a homogenous paste suitable for inclusion in a composition without disrupting said amalgamation. US 5 409 709 discloses a tablet composition comprising ibuprofen and paracetamol which is coated with a film coating formulation containing a glycerine fatty acid ester.

In addition, commercially available reference product of ibuprofen and paracetamol film coated tablet has the propensionof undergoing color change on the tablet surface during the storage in the presence of heat and moisture.Consequently, there is a need to develop formulation methods for preparing a stable combination of ibuprofen and paracetamol containing formulations.

Thus, the oral formulations disclosed in the prior art demand further improvements in light of the heat, moisture sensitive and metastable nature of ibuprofen. Also, improved stable oral formulations were yet desired to overcome the tendency of ibuprofen and paracetamol to undesirably exhibit color changes in tablet formulations along with the above-mentioned limitations.

The present invention provides a stabilized formulation of ibuprofen and paracetamol, possessing marked improvement in color change over shelf life employing a selective stabilizer for coating, which overcomes the drawbacks of processes recited in prior arts.

### Description of Invention:

An object of the present invention is to provide stabilized pharmaceutical tablet formulation of ibuprofen and paracetamol film tablet having color stability throughout its shelf-life, wherein said formulation is subcoated with a selective stabilizer.

According to the invention, a color stable pharmaceutical tablet formulation comprising; ibuprofen and paracetamol or pharmaceutically acceptable salts thereof as active ingredient, characterized in that said tablet comprises at least one coating layer comprising 0.1%-0.3% (w/w) in unit dose glyceryl monocaprylocaprate as stabilizer which is a medium chain glyceride of caprylic (8 carbon chain) and capric (10 carbon chain) mono- and diesters of glycerin mixtures.

The film tablet composition is provided in a unit dose formulation comprising 200 mg ibuprofen and 500 mg paracetamol.

For purposes of the present invention, the following terms are given further clarification as to their meanings, glycerin is synonymous with glycerol; and glycerol ester is synonymous with glyceride. Additionally, glyceryl monocaprylocaprate is synonymous with glyceryl monocaprylate/caprate; glyceryl caprylate/caprate; glycerol monocaprylocaprate;glycerides C8-10 mono-, di-, tri-; medium chain mono- & diglycerides; glyceryl mono- &dicaprylo/caprate; mono-diglycerides of caprylic/capric acid.

In the formulations of ibuprofen known to the art for the production of tablets, tablet color change has been noted. Tablets having a brilliant white color when freshly manufactured will deteriorate in appearance and acquire a brownishcolor in time. Further, the discoloration is exacerbated by elevated temperatures, and by moist environments. Although the discoloration phenomenon does not produce an increase in the number of total related substances and does not affect the therapeutic efficacy of the tablets,the browning and mottling appearance is not generally considered pharmaceutically acceptable for commercial purposes. Also;the aesthetic deterioration is such as to detract from the salability of the product. Hence, a tablet which is color stable for a greater length of time than that known to the art must be considered as a highly desirable product. It is the object of the present invention to provide a composition from which there may be produced tablets containing ibuprofen in combination with paracetamol having tablet color stability for a long time.

In the present invention, wet granulation is used instead of melt granulation. The major drawback of melt granulation is the need of high temperature during the process, which can cause degradation and/or oxidative instability of the ingredients, especially of the thermolabile drugs.

Tablet coating is one of the oldest pharmaceutical processes. There are many reasons for coating pharmaceutical tablets; some aesthetic, some functional. One important reason is to enhance drug stability; that is, to protect the drug from oxygen, moisture and light, the three key causes of drug degradation.

In general, the film-coating of the tablets is obtained with known techniques of coating in which the film-coating mixture, dissolved in water or in organic solvents, is applied with a procedure of continuous automatic spraying using a pressurized air flow which fixes the coating to the surface of the tablet. The coating, besides organoleptic (removal of unpleasant tastes or odors) or cosmetic reasons (improvement of color or aspect), is also adopted to protect the active ingredient contained in the tablet from atmospheric humidity and/or from light.

In this invention, use of film coating compositions comprising glyceryl monocaprylocaprate on oral tablets results in improved product appearance, for example, in terms of improved color stable tablets when compared to that of the prior art.

The inventive coating layer composition may contain further suitable excipients such as, but not limited, medium chain glycerides and optionally glidants, pigments, surfactants or other coating auxiliaries.

Medium chain triglycerides are high-chain (6 to 12 carbons) fatty acid esters of glycerol.They may be monoesters, diesters and triesters of glycerin with saturated aliphatic carboxylic acids having from 6 to 12 carbon atoms. Examples of such acids include straight chain acids such as hexanoic acid, heptanoic acid, octanoic acid (also known as caprylic acid), nonanoic acid and decanoic acid (also known as capric acid) as well as branched aliphatic carboxylic acids with a total carbon content of 6 to 12 atoms.

The esters useful in this invention preferably are derived from carboxylic acids containing from 6 to 12 carbon atoms. Particularly preferred are those esters derived from caprylic acid (8 carbon atoms). While mono, di, and tri-esters are all useful in this invention, monoesters are preferred. In addition, the ester may be part of a mixture comprising differing carbon atom content in the esters and/or comprising a mixture of monoglycerides, diglycerides, and triglycerides.

In most preferred aspects of the invention, the medium chain glycerides comprise a mixture of caprylic (8 carbon chain) and capric (10 carbon chain) mono- and diesters of glycerin. Medium chain triglycerides preferably used as stabilizers are selected from the group of hard fat in accordance with USP/NF, glycerol monooleate, glycerol monocaprylocaprate in accordance with the European Pharmacopeia (Ph. Eur.).

In this present invention, glyceryl monocapryiocaprate is used in the sub-coating layer. Glyceryl monocaprylocaprate Type I (also named as mono and diglycerides of caprylic/capric acid) is a mixture of monoacylglycerols, mainly mono-O-octanoylglycerol and mono-O-decanoylglycerol, containing variable quantities of di- and triacylglycerols, obtained by direct esterification of glycerol with caprylic (octanoic) and capric (decanoic) acids, followed by a distillation step in the case of glycerol motiocaprylocaprate (Type II). Glycerol monocaprylocaprate Type I comprise 45-75% monoesters, 20-50%, diesters, less than 10% triesters and less than 3% free glycerin. The carbon chain distribution is 50-90% C8 (or 8 carbons), 10-50% C10, less than 3% C12 and less than 1% C14. Another preferred glycerol monocaprylocaprate comprises 49-61% monoesters and 7% or less of free glycerin.

It has been discovered that such highly desirable color stability of the tablet compositions of the present invention is surprisingly achieved by applying a subcoating layer into the tablet with using glyceryl monocaprylocaprate. This coating layer prevents both morphologic changes of the outer surface (cuts, swellings or clefts) and degradation processes which can be easily identified by remarkable changes of color (from white to various shades of yellow).

Indeed, a suitable coating in this invention is capable to better protect the product and to delay during its storage the degradation processes which may be shown by possible changes of color of the tablet surface. On the other hand, it is fundamental that the used tablet coating does not affect the disintegration time of the tablet andbioavailability of the active ingredients.

The color stability of tablets prepared from the composition of the present invention has been compared to that of commercially available reference tablets. The color measurements were made visually and also with a colorimetric measuring instrument (type Datacolor 600, from the company Datacolor) for the so-called "b value", which is a measure of yellowing.

The color of the tablets was read to DataColor 600 which is an equipment used to compare the colors of the products.

According to Young-Hemlholtz theory of color vision, there are three receptors in the retina that are responsible for the perception of color and with different absorption spectra. One receptor is sensitive to green, another to blue and a third to the red. These colors can then be combined to form any visible color in the spectrum.

CIE (Commission International I'Eclaraige) generate Color Space in 1976 as like the Trichromatic Theory. There are different types of values to measure the color such as L*, a*, b*, H* and C* values. (L*: Lightness, a*: Green/Red axis, b*: Blue/Yellow axis, C*: Saturation, h: Shade)

Excipients are substances used in combination with an active ingredient to produce a pharmaceutical formulation. Some examples of excipients are binders, lubricants, disintegrants, diluents, colorants, flavors, glidants, surfactants, absorbants and sweetening agents.

The tablet composition according to present invention generally comprises 75 to 85 wt.% of active ingredients; 1 to 5 wt.% of wet granulation binder, 5 to 15 wt.% of diluents, 1 to 5 wt.% of disintegrant, 0.1 to 1 wt. % of glidant, 0.3 to 2 wt. %of lubricant, 0,1 to 0,3 wt. % of sub-coating layer and 1 to 5 wt. % of film coating agent.

In the framework of this invention, binders refer to excipients which enhance the linkage between particles. They include in a non-limiting manner, starch, gums, pregelatinized starch, polyvinyl pyrrolidone (PVP), copovidone, cellulose derivatives, such as hydroxypropylmethylcellulose, hydroxypropylcellulose, and carboxymethylcellulose and their salts. Advantageously, the binder involved in the process of the present invention and which comes into the composition of the final tablets is polyvinylpyrrolidone. Typically, the amount of binder within the scope of the invention is comprised, based on the total amount of the tablet, within the scope of 1 to 5% by weight.

Diluents intend to increase the bulk of the composition in order to facilitate the processing of tablets. Diluents within the scope of the invention comprise in addition to lactose, lactose monohydrate, mannitol, sucrose, maltodextrin, dextrin, maltitol, sorbitol, xylitol, powdered cellulose, cellulose gum, microcrystalline cellulose, starch, calcium phosphate, or metal carbonate. Typically, the diluent represent about 5 to about 95% by weight based on the total weight of the tablet, in this invention 5 to 15 % by weight.

The tablets according to the invention may further comprise disintegrants. Without being restrictive, the disintegrants within the scope of the invention include starch, croscarmellose sodium, crosspovidone, sodium starch glycolate and polacrilin potassium. Advantageously, the croscarmellose sodium disintegrant provides significant results in the dissolution rate of a tablet into the body after administration.

Typically, in this invention disintegrants represent about 1 to 5% by weight based on the total weight of the tablet.

Glidants may be useful in the early stages of the process of the invention in order to improve the flowability of the powder/granules before the compaction step. Thus, glidants may come into the composition of the tablets of the invention. Suitable glidants within the scope of the invention are, in a non-limiting manner, colloidal silicon, magnesium trisilicate, starch, talc and tribasic calcium phosphate. Glidants may advantageously be incorporated into the composition of the tablets of the invention in an amount comprised within the range of 0.1 to 1% by weight based on the total weight of the tablet.

Lubricants are intended to prevent adhesion of the tablet materials to the surface of diesand punches, reduce inter particle friction and may improve the rate of flow of the tablet granulation. Suitable lubricants are, in a non-limiting manner, talc, magnesium stearate, calcium stearate, polyethylene glycol, hydrogenated vegetable oils, stearic acid and sodium stearyl fumarate. Lubricants may advantageously be incorporated into the composition of the tablets of the invention in an amount comprised within the range of 0.3 to 2% by weight based on the total weight of the tablet.

According to invention, sub-coating layer is applied to prevent both morphologic changes of the outer surface and degradation processes. The tablets were sub-coated with glyceryl monocaprylocaprate. Sub-coating layer is incorporated into the composition of the tablets of the invention in an amount comprised within the range of 0.1 to 0.3% by weight based on the total weight of the tablet.

The film coating agent can be made from any commercially available powder mix for preparing coating suspensions. The coating may be prepared from the individual elements rather than from the commercially available preparation. Coating agents may advantageously represent 1 to 5% by weight based on the total weight of the tablet. The present invention is related to a color-stable pharmaceutical tablet formulation comprising; ibuprofen and paracetamol or pharmaceutically acceptable salts thereof, as defined in claim 1, wherein the process comprises the steps of:
a) Ibuprofen, paracetamol and one or more excipients are wet granulated,
b) the resulting granules are compressed into tablets,
c) the tablets are sub-coated with glyceryl monocaprylocaprate,
d) the resulting tablets are film coated.

The invention is illustrated in the following examples.

### Comparative Example 1.

| **Ibuprofen** - **Paracetamol 200 mg-500 mg Film Tablet** | **% amount in unit dose (%w/w)** |
|---|---|
| Ibuprofen | 24 |
| Paracetamol | 59 |
| Polyvinylpyrrolidone | 2.5 |
| Microcrystalline cellulose | 9.0 |
| Croscarmellose sodium | 4.1 |
| Colloidal silicon dioxide | 0.4 |
| Stearic acid | 0.5 |
| Magnesium stearate | 0.5 |
| PVA based film coating | q.s. |
| Deionized water | q.s. |

Ibuprofen, paracetamol, polyvinylpyrrolidone, microcrystalline cellulose, croscarmellose sodium and half of colloidal silicon dioxide are wet granulated with water. The producedcomposition was dried in a fluid-bed dryer and then milled. The milled material is blended with remaining colloidal silicon dioxide and stearic acid. Then, the final blend is obtained by adding magnesium stearate. Final blend is compressed into tablets and tablets are coated with polyvinyl alcohol based film coating material.

### Comparative Example 2.

| **Ibuprofen** - **Paracetamol 200 mg-500 mg Film Tablet** | **% amount in unit dose (%w/w)** |
|---|---|
| Ibuprofen | 24 |
| Paracetamol | 59 |
| Polyvinylpyrrolidone | 2.5 |
| Microcrystalline cellulose | 9.0 |
| Croscarmellose sodium | 4.1 |
| Colloidal silicon dioxide | 0.4 |
| Stearic acid | 0.5 |
| Magnesium stearate | 0.5 |
| Methacrylic acid copolymer | 0.1-0.3 |
| PVA based film coating | q.s. |
| Deionized water | q.s. |
| Ethanol | q.s. |

Ibuprofen, paracetamol, polyvinylpyrrolidone, microcrystalline cellulose, croscarmellose sodium and half of colloidal silicon dioxide are wet granulated with water. The wet material was dried in a fluid-bed dryer and then milled. The milled material is blended with remaining colloidal silicon dioxide and stearic acid. Then, the final blend is obtained by adding magnesium stearate. Final blend is compressed into tablets and tablets are subcoated with methacrylic acid copolymer. Finally tablets are recoated with polyvinyl alcohol based film coating material.

### Example 3.

| **Ibuprofen** - **Paracetamol 200 mg-500 mg Film Tablet** | **% amount in unit dose (%w/w)** |
|---|---|
| Ibuprofen | 24 |
| Paracetamol | 59 |
| Polyvinylpyrrolidone | 2.5 |
| Microcrystalline cellulose | 9.0 |
| Croscarmellose sodium | 4.1 |
| Colloidal silicon dioxide | 0.4 |
| Stearic acid | 0.5 |
| Magnesium stearate | 0.5 |
| Glyceryl monocaprylocaprate | 0.1-0.3 |
| PVA based film coating | q.s. |
| Deionized water | q.s. |
| Ethanol | q.s. |

Ibuprofen, paracetamol, polyvinylpyrrolidone, microcrystalline cellulose, croscarmellose sodium and half of colloidal silicon dioxide are wet granulated with water. The wet material was dried in a fluid-bed dryer and then milled. The milled material is blended with remaining colloidal silicon dioxide and stearic acid. Then, the final blend is obtained by adding magnesium stearate. Final blend is compressed into tablets and tablets are subcoated with glyceryl monocaprylocaprate. Finally tablets are recoated with polyvinyl alcohol based film coating material.

### Dissolution Profiles:

Comparative dissolution tests were conducted based on the general dissolution test method. The progress of dissolution is monitored for 30 minutes. Dissolution testing measures the portion (%) of ibuprofen and paracetamol that have been dissolved in the dissolution medium. Dissolution test was performed with USP Apparatus-II, pH 7.2 phosphate buffers, 50 rpm and 900 mL.The above exemplified compositions comprising ibuprofen and paracetamol were tested in vitro and they were compared with a commercial reference product.

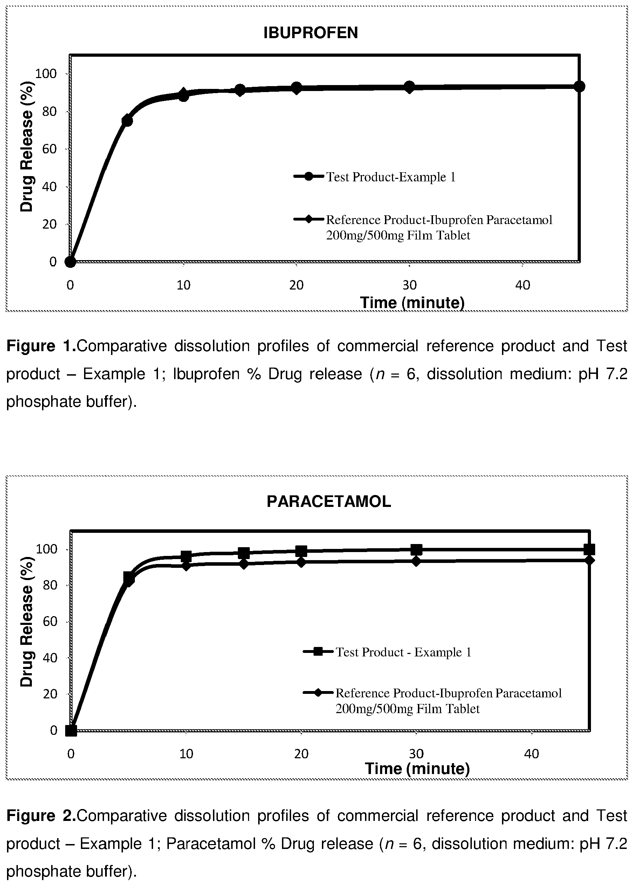

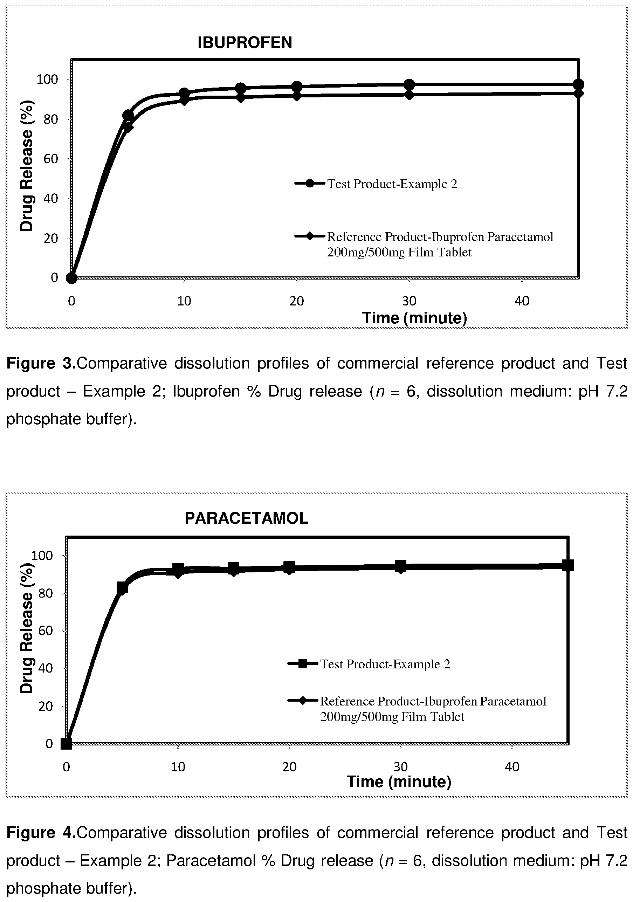

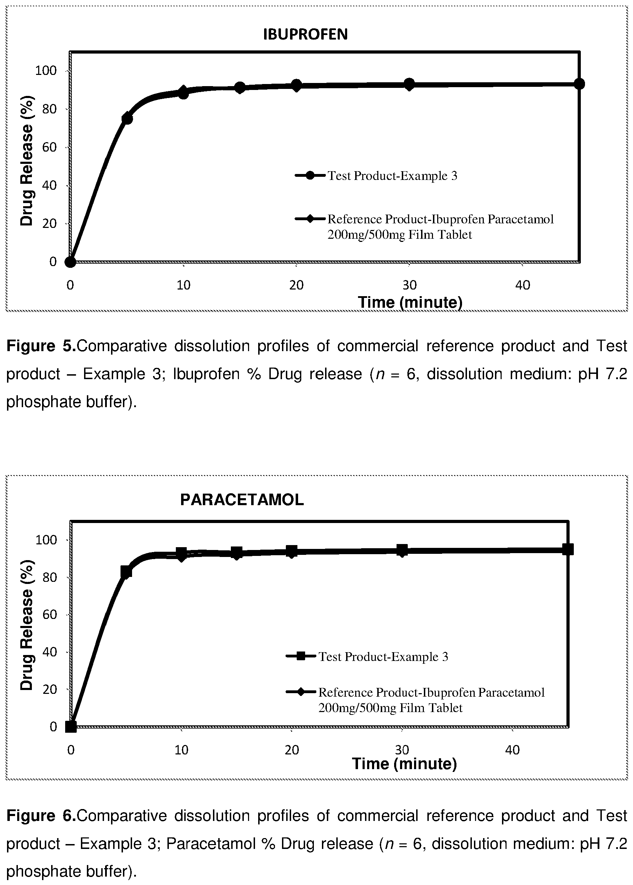

All the products tested were very rapidly dissolving (more than 85% of the labeled amount in 15 min), and the dissolution profiles of the Example1-3 were thus considered similar to that of the commercial reference product in pH 7.2 phosphate buffer. Figure 1-6 shows a comparative plot of the percentage of ibuprofen and paracetamol dissolved as a function of time in each of the test samples prepared by Example-1, Example-2 and Example-3 with commercial reference product.

Based on the drug release profiles (Figure 1-6), it has been proved that the content of the coating material does not affect dissolution rate profile.

### Stability Test

The stability of a drug substance is an important factor in the manufacture of safe and effective pharmaceutical products. Stability issues can be caused by environmental factors such as humidity, temperature and the like. The physical and chemical stability may be tested in conventional manner, e.g. the ibuprofen - paracetamol tablets may be tested as such by measurement of dissolution, friability, assay for both active substance, degradation products, and appearance.

Physical stability means that the formulation is totally unchanged throughout its shelf-life and has not suffered any changes by way of appearance, organoleptic properties (color, odor and taste), hardness, brittleness, particle size etc. Also, physical stability affects pharmaceutical elegance, drug content uniformity and drug release rate. The general appearance of a tablet, its visual identity and overall elegance is essential for consumer acceptance and lot-to-lot uniformity, general tablet-to-tablet uniformity, and for monitoring trouble free manufacturing. The control of general appearance of tablets involves the measurements of a number of tablet's size, shape, color, presence or absence of an odor, taste, surface texture, physical flaws and consistency, and legibility of any identifying markings.

In the development of ibuprofen - paracetamol tablets dosage form, stability was assessed under three different storage conditions (25°C ± 2°C, 30°C ± 2°C and 40°C ± 2°C) in temperature-programmable control cabinets. 25°C ± 2°C/ 60 ± 5% RH (Relative Humidity) represents long term condition and carries out up to 24 months; 30°C ± 2°C/ 65 ± 5% RH represents intermediate condition and carries out up to 12 months; 40°C ± 2°C/ 75 % ± 5 % RH represents accelerated conditions and carries out up to 6 months.

In order to reveal shelf life, stability test was performed to test products (Example 1-3) and reference product. Reference product and test product (Example 1-3) were kept at stability cabinets with same packaging material.

For the commercially available reference product, Example-1 and Example-2, the results showed that products kept at 25°C ± 2°C/ 60 ± 5% RH and 30°C ± 2°C/ 65 ± 5% RH show acceptable stability. However, when products kept at 40°C ± 2°C/75 % ± 5 % RH for 6 months, their colors changed dramatically. On the other hand; for the test products (Example 3), no physical and chemical change were occurred at all storage conditions, especially no color change at accelerated condition 40°C ± 2°C/75 % ± 5 % RH after 6 months.

Consequently, tablets of the present invention sub-coated with glyceryl monocaprylocaprate surprisingly exhibit a satisfactory rate of dissolution and an improved stability, since they do not undergo physical and chemical degradation processes which may be shown by changes of color, though stored even for long periods under long term and accelerated conditions.

During the stability study, the following parameters of the film coated tablet of the invention were assessed:

**Table 1.Comparative stability results after 6 months at 40°C ± 2°C/ 75 % ± 5 % RH storage condition**

| **Parameters** | **Limits** | **Reference product** | **Test product (Example 1)** | **Test product (Example 2)** | **Test product (Example 3)** |
|---|---|---|---|---|---|
| **Appearance (visually)** | White tablets | Yellow color(Brow nish) | Yellow color(Brownis h) | Yellow color(Brownis h) | White |
| **Dissolution** | **Ibuprofen:** | 92.6% | 92.0% | 96.4% | 93.9% |
| (900 ml, pH7.2, 50rpm, App II/ Paddle) | 30.min, 70%(Q) | | | | |
| | Paracetamol:30 | | | | |
| | .min, 70%(Q) | 93.9% | 99.8% | 94.2% | 94.5% |
| **Assay** | **Ibuprofen:** | 199.8 mg | 199.5 mg | 200.4 mg | 200.9 mg |
| | 200.0mg± 10% | | | | |
| | Paracetamol:50 | | | | |
| | 0.0mg± 10% | 500.6 mg | 498.9 mg | 500.4 mg | 499.8 mg |
| **Related Substance** | **Total impurity:** Not more than 0.5% | Comply | Comply | Comply | Comply |

In order to clarify the color change of the tablets, color of tablets prepared from the composition of the present invention (Test product - Example 3) has been measured and compared to that of commercially available reference tablets with a colorimetric measuring instrument (type Datacolor 600, from the company Datacolor).

**Table 2. Comparative Datacolor measurement results for Test product - Example 3 and reference products after 6 months at different stability conditions**

| **Sample** | **Stability condition** | **L** | **a** | **b** | **C** | **h** | **Gloss** |
|---|---|---|---|---|---|---|---|
| **Reference product** | 25°C ± 2°C/ 60% ± 5% RH | 93.62 | 0.61 | 4.5 | 4.54 | 82.29 | 39.536 |
| | 30°C ± 2°C/ 65% ± 5% RH | 91.85 | 1.1 | 5.69 | 5.8 | 79.1 | 36.934 |
| | 40°C ± 2°C/ 75% ± 5% RH | 90.27 | 1.55 | 6.99 | 7.16 | 77.48 | 25.93 |
| **Test product - Example 3** | 25°C ± 2°C/ 60% ± 5% RH | 96.71 | 0.33 | 1.2 | 1.24 | 74.56 | 3.642 |
| | 30°C ± 2°C/ 65% ± 5% RH | 95.69 | 0.51 | 1.48 | 2.01 | 74.6 | 3.841 |
| | 40°C ± 2°C/75% ± 5% RH | 95.47 | 0.65 | 2.5 | 2.58 | 75.46 | 4.0436 |

| | | | | | | | |
|---|---|---|---|---|---|---|---|
| (L*: Lightness, a*: Green/Red axis, b*: Blue/Yellow axis, C*: Saturation, h: Shade) | | | | | | | |

According to Datacolor results, the positive b values show that the tablets are more yellowish in color space. Reference tablets have pearlescent coating and test tablets have opaque white coating that's why gloss values of the reference tablets are higher than test tablets. It was concluded that color of the reference tablets get yellowish (especially brownish at 40°C ± 2°C/ 75% ± 5% RH) when the temperature increases.

In the following Table 3, Datacolor b values stability results for Test product - Example 3 and reference products are compared with respect to initial condition. According to results, there is a serious color change in reference tablets which shows higher delta b results in comparison with test tablets wherein delta b corresponds to the change in the b value relative to the initial value.

**Table 3. Comparative Datacolor b value results for Test product - Example 3 and reference products initially and after 6 months at 40°C ± 2°C/ 75 % ± 5 % RH storage condition**

| **Parameters** | **Initial** | | **After** 6 **months** | |
|---|---|---|---|---|
| | **Reference product** | **Test product (Example 3)** | **Reference product** | **Test product (Example 3)** |
| | **b value** | **b value** | **b value** | **b value** |
| **Appearance (Datacolor)** | 1.60 | 1.15 | 6.99 | 2.50 |

As demonstrated above, the test product prepared by Example 3 is stable at 40°C ± 2°C/75 % ± 5 % RH for 6 months in contrast to commercially available reference product. The formulation of the present invention advantageously provides color stable ibuprofen - paracetamol film tablet products which are resistant to the undesirable color change frequently observed in prior art ibuprofen-containing formulations. This situation exhibit improved shelf-life stability for ibuprofen-paracetamol film tablets.

### Explanation of figures:

**Figure 1****.**Comparative dissolution profiles of commercial reference product and Test product - Example 1; Ibuprofen % Drug release (*n* = 6, dissolution medium: pH 7.2 phosphate buffer).
**Figure 2****.**Comparative dissolution profiles of commercial reference product and Test product - Example 1; Paracetamol % Drug release (*n* = 6, dissolution medium: pH 7.2 phosphate buffer).
**Figure 3****.**Comparative dissolution profiles of commercial reference product and Test product - Example 2; Ibuprofen % Drug release (*n* = 6, dissolution medium: pH 7.2 phosphate buffer).
**Figure 4**.Comparative dissolution profiles of commercial reference product and Test product - Example 2; Paracetamol % Drug release (*n* = 6, dissolution medium: pH 7.2 phosphate buffer).
**Figure 5****.**Comparative dissolution profiles of commercial reference product and Test product - Example 3; Ibuprofen % Drug release (*n* = 6, dissolution medium: pH 7.2 phosphate buffer).
**Figure 6****.**Comparative dissolution profiles of commercial reference product and Test product - Example 3; Paracetamol % Drug release (*n* = 6, dissolution medium: pH 7.2 phosphate buffer).

## Claims

1. A pharmaceutical tablet formulation comprising; ibuprofen and paracetamol or a pharmaceutically acceptable salts thereof as active ingredient, **characterized in that** said tablet comprises a sub-coating layer wherein this layer comprising a high-chain (6 to 12 carbons) fatty acid esters of glycerol which is glyceryl monocaprylocaprate with a content of 0.1%-0.3% (w/w) by weight of the tablet composition.

2. The formulation according to claim 1, wherein ibuprofen and paracetamol are granulated together or seperately with one or more pharmaceutically acceptable excipients by wet granulation method.

3. The formulation according to claim 1, wherein said product obtainable by process which is defined as;
a) Ibuprofen, paracetamol and one or more excipients are wet granulated,
b) the resulting granules are compressed into tablets,
c) the tablets are sub-coated with glyceryl monocaprylocaprate,
d) the resulting tablets are film coated.

4. The formulation according to claim 3, wherein said product comprises;
a) 75 to 85 wt.% of active ingredients as ibuprofen and paracetamol or pharmaceutically acceptable salts,
b) 1 to 5 wt.% of wet granulation binder,
c) 5 to 15 wt.% of diluents,
d) 1 to 5 wt.% of disintegrant,
e) 0,1 to 1 wt.% of glidant,
f) 0,3 to 2 wt.% lubricant,
g) 0,1 to 0,3 wt.% sub-coating layer,
h) 1 to 5 wt.% film coating.

5. The formulation according to claim 4, wherein binders used in said product are selected from the group consisting of starch, gums, pregelatinized starch, polyvinyl pyrrolidone (PVP), copovidone, cellulose derivatives, such as hydroxypropylmethylcellulose, hydroxypropylcellulose, and carboxymethylcellulose and their salts.

6. The formulation according to claim 4, wherein diluents used in said product are selected from the group consisting of lactose, lactose monohydrate, mannitol, sucrose, maltodextrin, dextrin, maltitol, sorbitol, xylitol, powdered cellulose, cellulose gum, microcrystalline cellulose, starch, calcium phosphate, or metal carbonate.

7. The formulation according to claim 4, wherein disintegrants used in said product are selected from the group consisting of starch, croscarmellose sodium, crosspovidone, sodium starch glycolate and polacrilin potassium.

8. The formulation according to claim 4, wherein glidants used in said product are selected from the group consisting of colloidal silicon, magnesium trisilicate, starch, talc and tribasic calcium phosphate.

9. The formulation according to claim 4, wherein lubricants used in said product are selected from the group consisting of talc, magnesium stearate, calcium stearate, polyethylene glycol, hydrogenated vegetable oils, stearic acid, sodium stearyl fumarate.

## Patentansprüche

1. Eine pharmazeutische Tablettenformulierung, umfassend: Ibuprofen und Paracetamol oder ein pharmazeutisch akzeptables Salz davon als Wirkstoff, **dadurch gekennzeichnet, dass** die Tablette eine Unterschicht umfasst, wobei diese Schicht einen hochkettigen (6 bis 12 Kohlenstoffatome) Fettsäureester von Glycerin umfasst, bei dem es sich um Glycerylmonocaprylocaprat handelt von 0,1-0,3 % (Gew./Gew.) der Tablettenzusammensetzung.

2. Formulierung nach Anspruch 1, wobei Ibuprofen und Paracetamol zusammen oder getrennt mit einem oder mehreren pharmazeutisch verträglichen Hilfsstoffen durch ein Nassgranulationsverfahren granuliert werden.

3. Formulierung nach Anspruch 1, wobei das Produkt durch ein Verfahren erhältlich ist, das definiert ist als:
a) Ibuprofen, Paracetamol und ein oder mehrere Hilfsstoffe werden nass granuliert,
b) das resultierende Granulat wird zu Tabletten verpresst,
c) mit Glycerylmonocaprylocaprat beschichtet.
d) Die resultierenden Tabletten sind mit einem Film überzogen.

4. Formulierung nach Anspruch 3, wobei das Produkt Folgendes umfasst:
a) 75 bis 85 Gew.-% Wirkstoffe wie Ibuprofen und Paracetamol oder pharmazeutisch verträgliche Salze,
b) 1 bis 5 Gew.-% Nassgranulationsbindemittel,
c) 5 bis 15 Gew.-% Verdünnungsmittel,
d) 1 bis 5 Gew.-% Sprengmittel,
e) 0,1 bis 1 Gew.-% Gleitmittel,
f) 0,3 bis 2 Gew.-% Gleitmittel,
g) 0,1 bis 0,3 Gew.-% Grundierungsschicht,
h) 1 bis 5 Gew.-% Filmbeschichtung.

5. Formulierung nach Anspruch 4, wobei die in dem Produkt verwendeten Bindemittel aus der Gruppe ausgewählt sind, die aus Stärke, Gummi, vorverkleisterter Stärke, Polyvinylpyrrolidon (PVP), Copovidon, Cellulosederivaten wie Hydroxypropylmethylcellulose, Hydroxypropylcellulose und Carboxymethylcellulose und deren Salzen besteht.

6. Formulierung nach Anspruch 4, wobei die in dem Produkt verwendeten Verdünnungsmittel aus der Gruppe ausgewählt sind, die aus Lactose, Lactose-Monohydrat, Mannitol, Saccharose, Maltodextrin, Dextrin, Maltitol, Sorbitol, Xylitol, pulverisierter Cellulose, Cellulosegummi, mikrokristalliner Cellulose, Stärke, Calciumphosphat oder Metallcarbonat.

7. Formulierung nach Anspruch 4, wobei die in dem Produkt verwendeten Sprengmittel aus der Gruppe bestehend aus Stärke, Croscarmellose-Natrium, Crosspovidon, Natriumstärkeglykolat und Polacrilin-Kalium ausgewählt sind.

8. Formulierung nach Anspruch 4, wobei die in dem Produkt verwendeten Gleitmittel aus der Gruppe bestehend aus kolloidalem Silicium, Magnesiumtrisilicat, Stärke, Talk und tribasischem Calciumphosphat ausgewählt sind.

9. Formulierung nach Anspruch 4, wobei die in dem Produkt verwendeten Gleitmittel aus der Gruppe ausgewählt sind, die aus Talk, Magnesiumstearat, Calciumstearat, Polyethylenglykol, hydrierten Pflanzenölen, Stearinsäure und Natriumstearylfumarat besteht.

## Revendications

1. Comprimé pharmaceutique comprenant de l'ibuprofène et du paracétamol ou leurs sels pharmaceutiquement acceptables en tant qu'ingrédients actifs, **caractérisé par le fait que** ledit comprimé comprend une couche de sous-enduction dans laquelle cette couche comprend un ester d'acide gras à chaîne élevée (6 à 12 carbones) de glycérol qui est le monocaprylocaprate de glycéryle avec une teneur de 0,1 % à 0,3 % (p/p) par rapport au poids de la composition du comprimé.

2. Formulation selon la revendication 1, dans laquelle l'ibuprofène et le paracétamol sont granulés ensemble ou séparément avec un ou plusieurs excipients pharmaceutiquement acceptables par une méthode de granulation humide.

3. Formulation selon la revendication 1, dans laquelle ledit produit peut être obtenu par un procédé défini comme suit;
a) L'ibuprofène, le paracétamol et un ou plusieurs excipients sont granulés par voie humide,
b) les granulés obtenus sont compressés en comprimés,
c) les comprimés sont enrobés de glycéryl monocaprylocaprate,
d) les comprimés obtenus sont recouverts d'un film.

4. Formulation selon la revendication 3, dans laquelle ledit produit comprend ;
a) 75 à 85 % en poids d'ingrédients actifs tels que l'ibuprofène et le paracétamol ou des sels pharmaceutiquement acceptables,
b) 1 à 5 % en poids de liant de granulation humide,
c) 5 à 15 % en poids de diluants,
d) 1 à 5 % en poids de désintégrant,
e) 0,1 à 1 % en poids d'agent de glaçage,
f) 0,3 à 2 % en poids de lubrifiant,
g) couche de sous-couche de 0,1 à 0,3 % en poids,
h) 1 à 5 % en poids de revêtement de film.

5. Formulation selon la revendication 4, dans laquelle les liants utilisés dans ledit produit sont choisis dans le groupe constitué par l'amidon, les gommes, l'amidon prégélatinisé, la polyvinylpyrrolidone (PVP), la copovidone, les dérivés de la cellulose, tels que l'hydroxypropylméthylcellulose, l'hydroxypropylcellulose et la carboxyméthylcellulose, et leurs sels.

6. Formulation selon la revendication 4, dans laquelle les diluants utilisés dans ledit produit sont choisis dans le groupe constitué par le lactose, le lactose monohydraté, le mannitol, le saccharose, la maltodextrine, la dextrine, le maltitol, le sorbitol, le xylitol, la cellulose en poudre, la gomme de cellulose, la cellulose microcristalline, l'amidon, le phosphate de calcium ou le carbonate de métal.

7. Formulation selon la revendication 4, dans laquelle les désintégrants utilisés dans ledit produit sont choisis dans le groupe constitué par l'amidon, la croscarmellose sodique, la crosspovidone, le glycolate d'amidon sodique et la polacriline potassique.

8. Formulation selon la revendication 4, dans laquelle les agents de glissement utilisés dans ledit produit sont choisis dans le groupe constitué par le silicium colloïdal, le trisilicate de magnésium, l'amidon, le talc et le phosphate de calcium tribasique.

9. Formulation selon la revendication 4, dans laquelle les lubrifiants utilisés dans ledit produit sont choisis dans le groupe constitué par le talc, le stéarate de magnésium, le stéarate de calcium, le polyéthylène glycol, les huiles végétales hydrogénées, l'acide stéarique, le fumarate de stéaryle sodique.
